# EUROPÄISCHE PATENTSCHRIFT

(11) **EP 3 929 176 B1**
(45) Veröffentlichungstag und Bekanntmachung des Hinweises auf die Patenterteilung: **28.12.2022**
(21) Anmeldenummer: 20182396.0
(22) Anmeldetag: 25.06.2020
(51) Int. Cl.: C07C 215/68, C07C 217/50, C08G 18/32, C08G 18/50, C08G 59/50, C08G 65/26

(54) **FLÜSSIGE MISCHUNGEN PROPOXYLIERTER PARA-TOLUIDINE**
LIQUID MIXTURES OF PROPOXYLATED PARA-TOLUIDINES
MÉLANGES LIQUIDES DE PARA-TOLUIDINES PROPOXYLÉES

(43) Veröffentlichungstag der Anmeldung: 29.12.2021
(73) Patentinhaber: Saltigo GmbH, 51369 Leverkusen (DE)
(72) Erfinder: HÖFNER, THOMAS, 40764 Langenfeld (DE)
(74) Vertreter: Matzke, Michael

(56) Entgegenhaltungen:
- EP-A2- 2 518 033
- DE-A1-102004 042 858

## Beschreibung

Die Erfindung betrifft Mischungen propoxylierter 4-Toluidine (para-Toluidine) enthaltend zwei oder mehr verschiedene di- oder tripropoxylierte oder höher propoxylierte p-Toluidine in spezifischen Mengenverhältnissen, Verfahren zu deren Herstellung sowie deren Verwendung als Polymerisationsbeschleuniger oder als Vulkanisationsbeschleuniger oder als Härterkomponente für Epoxidharze.

Vernetzte Polymere können durch radikalische Polymerisation erzeugt werden. Dabei werden beispielsweise ungesättigte Polyester eingesetzt. Der Polymerisationsprozess wird auch als Härtung (curing) bezeichnet. Die Polymerisation wird für diese Gruppe von Polymeren durch sogenannte Härter (curing agents) eingeleitet. Dies sind in der Regel Radikalinitiatoren, beispielsweise Peroxide. Der bekannteste und am breitesten eingesetzte Härter ist Dibenzoylperoxid. Dabei werden auch häufig Polymerisationsbeschleuniger (accelerators) eingesetzt, die den Polymerisationsprozess beschleunigen und dabei den Härtungsprozess und/oder die Produkteigenschaften des Polymers vorteilhaft beeinflussen. Einige Polymerisationsbeschleuniger können dabei vorteilhafterweise über weitere funktionelle Gruppen in das Polymer eingebaut werden. Tertiäre Amine in Form N,N-disubstituierter Toluidine stellen eine wichtige Gruppe solcher Polymerisationsbeschleuniger dar - darunter wegen ihrer geringen Flüchtigkeit und ihres vorteilhafteren toxikologischen Profils insbesondere die Gruppe der ethoxylierten und propoxylierten Toluidine.

Die Einzelverbindung N,N-Bis-(2-hydroxypropyl)-p-toluidin [N,N-Bis-(2-hydroxypropyl)-4-toluidin, N,N-Dipropoxy-p-toluidin, 1,1'-(p-Tolylimino)dipropan-2-ol, CAS RN 38668-48-3; engl.: Diisopropanol-p-toluidine, N,N-Di(2-hydroxypropyl)-p-toluidine], also "dipropoxyliertes" para-Toluidin, ist bekannt. Dessen höhere Homologe sind bisher nur generisch offenbart.

RU2063960A beschreibt ethoxylierte p-Toluidine und spezifisch deren Herstellung aus 4-Toluidin und 3 bis 4 mol Ethylenoxid pro eingesetztem mol 4-Toluidin bei 80 ± 5 °C ohne den Zusatz von Lösungsmitteln und ohne den Zusatz von Katalysatoren. Dabei werden flüssige Mischungen von ethoxylierten 4-Toluidinen erhalten, deren Zusammensetzung nicht weiter beschrieben ist. Von den chemischen Ausbeuten lässt sich ableiten, dass der durchschnittliche Grad der Ethoxylierung (Anzahl der Ethylenoxideinheiten pro Molekül 4-Toluidin) zwischen 2 und 2,5 liegt. Über die Verteilung der einzelnen Homologe ist nichts bekannt.

EP 1650184 A1 beschreibt generisch Homologe von N,N-Bis-(2-hydroxyalkyl)-p-toluidin, die weniger als 0,2 Gew.% alkoxylierte 3-Toluidine, bezogen auf alkoxylierte 4-Toluidine, enthalten. Spezifisch wird deren Herstellung aus 4-Toluidin, das weniger als 0,2 Gew.% 3-Toluidin enthält, und 2,2 bis 5 mol, bevorzugt 2,3 bis 4 mol, besonders bevorzugt 2,3 bis 3,5 mol und insbesondere 2,5 bis 2,6 mol Alkylenoxid pro mol 4-Toluidin, offenbart.

Beispielsweise wurde bei der Umsetzung von 4-Toluidin, das weniger als 0,2 Gew.% 3-Toluidin, bezogen auf 4-Toluidin, enthält, mit 2,5 mol Ethylenoxid pro eingesetztem mol 4-Toluidin ohne den Zusatz von Lösungsmitteln und ohne den Zusatz von Katalysatoren bei 120 °C ein ethoxyliertes 4-Toluidin erhalten, das kein 4-Toluidin mehr enthält (Nachweisgrenze: 100 ppm), und eine Mischung aus < 0,1 % N-Hydroxyethyl-4-toluidin, 50,1 % N,N-Bis-(hydroxyethyl)-4-toluidin sowie 43,7 % N-Oxyethyl-N-(hydroxyethyloxyethylen)-4-toluidin, 5,4 % Tetraoxyethyl-4-toluidin, 0,7 % Pentaoxethyl-4-toluidin und einer Spur Hexaaoxethyl-4-toluidin darstellte.

Bei der Umsetzung von 4-Toluidin, das weniger als 0,2 Gew.% 3-Toluidin, bezogen auf 4-Toluidin, enthält, mit 2,58 mol Ethylenoxid pro eingesetztem mol 4-Toluidin ohne den Zusatz von Lösungsmitteln und mit 30 %iger Natriummethylatlösung als Katalysator wurde bei 120 °C ein ethoxyliertes 4-Toluidin erhalten, das Mischungen von 47,4 % N,N-Bis-(hydroxyethyl)-4-toluidin, 43,4% N-Oxyethyl-N-(hydroxyethyloxyethyl)-4-toluidin sowie Spuren höher oxethylierter Verbindungen darstellte. Das Dokument enthält die Beschreibung weiterer Beispiele, zu denen die Verteilung der Homologe jedoch nicht bekannt ist.

Da die Reaktion von 4-Toluidin mit Ethylenoxid, zumindest unter Verwendung eines Katalysators, nahezu quantitativ verläuft, lässt sich basierend auf den chemischen Massenausbeuten abschätzen, dass der durchschnittliche Grad der Ethoxylierung (die Summe aus m und n in allgemeiner Formel (I) in dem Dokument) nahe an dem der molaren Verhältnisse zwischen Ethylenoxid und 4-Toluidin liegt.

N,N-Bis-(hydroxyethyl)-4-toluidin (CAS RN 3077-12-1) wird von der Firma LANXESS Deutschland GmbH / Saltigo GmbH als schwach bis gelbbraun gefärbtes, flüssiges bzw. erstarrtes Produkt für die Anwendung als Härterkomponente für Epoxidharze vertrieben.

Ebenfalls wird von der Firma LANXESS Deutschland GmbH / Saltigo GmbH ein "überethoxyliertes N,N-Bis-(hydroxyethyl)-4-toluidin", das weniger als 0,2 Gew.% ethoxyliertes 3-Toluidin enthält, mit dem Namen Beschleuniger PT25E/2 als farblose bis schwach gelblich-bräunliche, viskose Flüssigkeit für die Anwendung als Härterkomponente für Epoxidharze vertrieben.

CN101200432 A offenbart das einfachere N,N-Bis-(hydroxpropyl)-anilin und dessen höhere Homologe. Diese werden aus der Umsetzung von N,N-Bis-(hydroxpropyl)-anilin in Gegenwart von 3 bis 3,6 mol Ethylenoxid pro eingesetztem mol Anilin bei Temperaturen von 145 bis 165 °C in Gegenwart von Katalysatoren wie Alkalimetallhydroxide oder gemischte Metallcyanide erhalten.

N,N-Bis-(2-hydroxpropyl)-p-toluidin (CAS RN 38668-48-3) ist bekannt und wird unter anderem von der Firma LANXESS Deutschland GmbH / Saltigo GmbH als schwach gelbe, erstarrte Schmelze für die Anwendung als Härterkomponente für Epoxidharze vertrieben.

Je nach Einsatzgebiet und Epoxidharzsystem sind erfahrungsgemäß einmal die ethoxylierten Aniline, die ethoxylierten Toluidine oder das N,N-Bis-(2-hydroxpropyl)-p-toluidin vorteilhafter. N,N-Bis-(2-hydroxpropyl)-p-toluidin hat jedoch den Nachteil, dass es bei Raumtemperatur in Form einer erstarrte Schmelze vorliegt, sodass das N,N-Bis-(2-hydroxpropyl)-p-toluidin vor Anwendung durch Erwärmen des Gebindes, das das Produkt enthält, geschmolzen werden muss. Es hat sich dabei gezeigt, dass verschiedene Isomere des N,N-Bis-(2-hydroxpropyl)-p-toluidins unterschiedliche Schmelzpunkte aufweisen. Dies führte dazu, dass sich beim teilweise Aufschmelzen des Produktes die niedriger schmelzenden Isomere in der flüssigen Phase anreicherten und nach Entnahme des bereits verflüssigten Anteils der Schmelzpunkt des verbleibenden Feststoffes immer weiter anstieg. Insofern handelte es sich bei diesem Phänomen um eine Art "unbeabsichtigtes Melt Refining". Wollte man dieses Phänomen vermeiden, müsste man N,N-Bis-(2-hydroxpropyl)-p-toluidin auf deutlich höhere Temperaturen als beispielsweise N,N-Bis-(hydroxyethyl)-p-toluidin aufheizen, was einen anwendungstechnischen Nachteil darstellt. Dadurch, dass das mittlere Kohlenstoffatom im als Edukt zur Herstellung eingesetzten Propylenoxid asymmetrisch ist und zwei Moleküle R- oder S-Propylenoxid mit einem Molekül 4-Toluidin reagieren, liegt N,N-Bis-(2-hydroxpropyl)-p-toluidin als Gemisch verschiedener Isomeren, bspw. RR, SS, oder meso vor. Zusätzlich kann der Epoxidring durch den Stickstoff des 4-Toluidins an der terminalen CH₂-Gruppe, was überwiegend erfolgt, oder an der mittleren CH-Gruppe angegriffen und geöffnet werden.

A. Zoltanski; et al., Current Applied Polymer Science, 2018, 2 (2), 89-93, The Structure of Propoxylated p-Toluidine, Used as a Polymerization Accelerator or in Unsaturated Polyester Resin Curing, haben die unterschiedlichen Strukturen, die bei der Reaktion von 4-Toluidin mit zwei Molekülen racemischem Propylenoxid entstehen, aufgeklärt.

Durch eigene Analysen der unterschiedlich schmelzenden Fraktionen des N,N-Bis-(2-hydroxpropyl)-p-toluidin wurde festgestellt, dass beispielsweise das optisch inaktive meso-Isomer bei höherer Temperatur schmilzt als die optisch aktiven Isomeren. Damit weist N,N-Bis-(2-hydroxpropyl)-p-toluidin den technischen Nachteil auf, dass es als komplexes Gemisch in Form einer erstarten Schmelze vorliegt und damit nur aufwändig als homogene Mischung der einzelnen Komponenten in die technische Anwendung eingebracht werden kann.

Es bestand daher die technische Aufgabe, eine Form des propoxylierten 4-Toluidins bereitzustellen, das den Nachteil des N,N-Bis-(2-hydroxpropyl)-p-toluidins nicht aufweist, aber in den Polymersystemen, in denen N,N-Bis-(2-hydroxpropyl)-p-toluidins eingesetzt werden kann, zumindest genauso gut oder besser als Polymerisationsbeschleuniger oder als Vulkanisationsbeschleuniger oder als Härterkomponente für Epoxidharze verwendet werden kann.

Diese Aufgabe wurde überraschenderweise gelöst durch die Bereitstellung von
Mischungen enthaltend zwei oder mehr verschiedene Verbindungen der allgemeinen Formel (I), 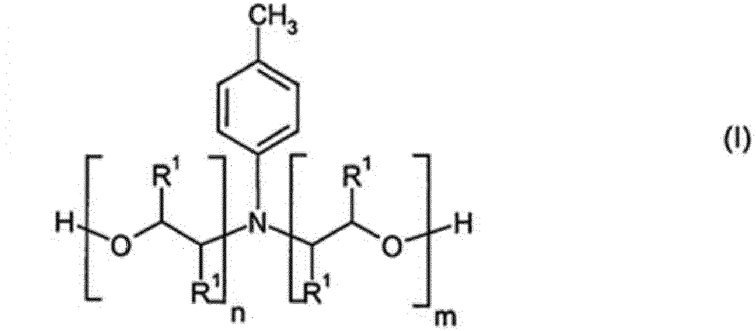 worin R¹ Wasserstoff oder Methyl bedeutet, wobei aber die Reste R¹, die sich an direkt benachbarten Kohlenstoffatomen befinden, nicht gleichzeitig Wasserstoff und nicht gleichzeitig Methyl und m und n ganze Zahlen darstellen, dadurch gekennzeichnet, dass
4-Toluidin in einem Anteil von bis maximal 2 Gew.%, bevorzugt von 0,001 bis 1 Gew.%, bezogen auf die gesamte Masse aller Verbindungen der Formel (I) in der Mischung vorliegt, und

die Verbindungen der Formel (I), bei denen die Summe aus m und n die ganze Zahl 2 beträgt, in einem Anteil von maximal 20 Gew.%, bevorzugt von 0,01 bis 20 Gew.%, besonders bevorzugt von 0,01 bis 12 Gew.%, bezogen auf die gesamte Masse aller Verbindungen der Formel (I) in der Mischung vorliegen, und

die Verbindungen der Formel (I), bei denen die Summe aus m und n mindestens die ganze Zahl 6 beträgt, in einem Anteil von maximal 40 Gew.%, bevorzugt von 0,01 bis 40 Gew.%, besonders bevorzugt von 0,01 bis 20 Gew.%, bezogen auf die gesamte Masse aller Verbindungen der Formel (I) in der Mischung vorliegen.

Gegenstand der Erfindung sind somit die erfindungsgemäßen Mischungen enthaltend zwei oder mehr verschiedene Verbindungen der Formel (I). Durch die Formulierung "enthaltend zwei oder mehr verschiedenen Verbindungen" ist ausgeschlossen, dass ausschließlich ein Homologes wie N,N-Bis-(2-hydroxpropyl)-p-toluidin oder N,N-Bis-(2-hydroxypropyloxypropylen)-p-toluidin vorliegt. Die Verbindungen der Formel (I), bei denen die Summe aus m und n identisch ist, stellen pro Summe aus m und n jeweils ein Homologes des N,N-Bis-(2-hydroxpropyl)-p-toluidins dar. Als Homologe werden somit Verbindungen der Formel (I) bezeichnet, in denen die Gesamtanzahl der Oxypropylen-Einheiten unterschiedlich ist.

Die entsprechende Verteilung des Propoxylierungsgrades lässt sich beispielsweise durch GC-MS ermitteln. Durch Kalibrierung der GC-Auswertungen mittels Kalibiersubstanzen können Bestimmungen von Gewichtsprozenten auch mit Gaschromatographie erfolgen.

Bevorzugt enthalten die erfindungsgemäßen Mischungen die Verbindungen der Formel (I), bei denen die Summe aus m und n die ganze Zahl 3 beträgt, in einem Anteil von 7 bis 49 Gew.%, besonders bevorzugt von 15 bis 49 Gew.%, bezogen auf die gesamte Masse aller Verbindungen der Formel (I) in der Mischung.

Ebenfalls bevorzugt enthalten die erfindungsgemäßen Mischungen die Verbindungen der Formel (I), bei denen die Summe aus m und n die ganze Zahl 4 beträgt, in einem Anteil von 10 Gew.% bis 49 Gew.%, besonders bevorzugt von 10 bis 40 Gew.%, bezogen auf die gesamte Masse aller Verbindungen der Formel (I) in der Mischung.

In einer weiteren bevorzugten Ausführungsform weist in den erfindungsgemäßen Mischungen jede homologe Gruppe von Verbindungen der Formel (I) in der Mischung in einem Anteil von kleiner als 50 Gewichtsprozenten bezogen auf die gesamte Masse aller Verbindungen der Formel (I) in der Mischung auf. Als homologe Gruppe von Verbindungen der Formel (I) wird jede Gruppe von Verbindungen mit identischer Summe aus m und n bezeichnet, die sich jedoch in der Kombination aus m und n unterscheiden können. Beispielsweise gehören zu der Gruppe der homologen Verbindungen mit der Summe aus m und n gleich 4 die Verbindungen mit
- m gleich 0 und n gleich 4 bzw. m gleich 4 und n gleich 0,
- m gleich 1 und n gleich 3 bzw. m gleich 3 und n gleich 1 und
- m gleich n gleich 2
sowie die einzelnen Isomere der Verbindungen mit den oben genannten Werten für m und n.

Dies hat den Vorteil, dass das Stoffgemisch in bestimmten Regionen oder Ländern chemikalienrechtlich als Polymer eingestuft wird und damit anderen chemikalienrechtlichen Bedingungen als Einzelsubstanzen unterliegt.

In einer ebenfalls bevorzugten Ausführungsform weisen die erfindungsgemäßen Mischungen einen Anteil an 4-Toluidin von weniger als 0,1 Gew.% bezogen auf die gesamte Masse aller Verbindungen der Formel (I) in der Mischung auf.

Die erfindungsgemäßen Mischungen können neben den Verbindungen der Formel (I) auch noch weitere Bestandteile enthalten. Dies können Reste von Katalysatoren, Wasser oder andere Polymerisationsprodukte von Propylenoxid sein. Die Summe der Gewichtsprozente aller Verbindungen der Formel (I) und der Gewichtsprozente der weiteren Bestandteile addieren sich zu 100 Gewichtsprozent. Typischerweise enthalten die erfindungsgemäßen Mischungen von 96 bis 100 Gew.% Verbindungen der Formel (I).

Die erfindungsgemäßen Mischungen weisen üblicherweise bei Raumtemperatur und/oder bei Temperaturen von 5 bis 40 °C einen flüssigen Aggregatzustand auf. Daneben weisen die erfindungsgemäßen Mischungen vorzugsweise keine festen Bestandteile auf. Bevorzugt sind die erfindungsgemäßen Mischungen keine Suspensionen. Die erfindungsgemäßen Mischungen können somit bevorzugt als homogene Flüssigkeiten bei Umgebungstemperatur gehandhabt werden. Dies hat den Vorteil, dass die erfindungsgemäßen Mischungen für die Anwendung als Polymerisationsbeschleuniger oder als Vulkanisationsbeschleuniger oder als Härterkomponente einfach, in exakten Mengen und in stabiler definierter Zusammensetzung dem Gebinde in flüssigem Zustand entnommen und in exakten Mengen und in stabiler definierter Zusammensetzung in die Anwendung eingesetzt werden können. Erfindungsgemäß weisen die Mischungen einen flüssigen Aggregatzustand auf, wenn sie bei 25 °C eine dynamische Viskosität von 0,1 bis 20000 mPas (Millipascalsekunden) aufweisen.

Zur Messung der dynamischen Viskosität können unterschiedliche Verfahren verwendet werden, beispielsweise Kapillar- oder Rotationsviskosimeter. Sofern nicht anders angegeben, wurden die dynamische Viskositäten gemäß DIN 53019 mit einem Rotationsviskosimeter nach dem Prinzip des Kegel-Platte-Messsystems (vgl. DIN 53019-2, Kapitel 10.3) bei den angegebenen Temperaturen gemessen. Die erfindungsgemäßen Mischungen weisen bevorzugt eine dynamische Viskosität von 500 bis 20000 mPas bei einer Temperatur von 25 °C, gemessen nach DIN 53019 mit einem Rotationsviskosimeter, auf.

Die erfindungsgemäßen Mischungen stellen die direkt aus dem erfindungsgemäßen Herstellungsverfahren resultierenden Produkte dar.

Gegenstand der Erfindung sind demnach auch die nach dem erfindungsgemäßen Verfahren erhältlichen erfindungsgemäßen Mischungen.

Die erfindungsgemäßen Mischungen können überraschend mit einem einfachen und stabilem Verfahren hergestellt werden. Bei einer Nachweisgrenze per Gaschromatographie von 100 ppm kann bevorzugt in den erfindungsgemäßen Mischungen kein nicht-umgesetztes 4-Toluidin mehr nachgewiesen werden. Dies ist von wesentlicher Bedeutung, da die am Stickstoff unalkylierten Toluidine starke Blutgifte und als krebserregend eingestuft sind.

Dieses erfindungsgemäße Verfahren zur Herstellung der Mischungen umfasst die Umsetzung der Verbindungen der Formel (I), in der R¹ Wasserstoff oder Methyl bedeuten, wobei aber die Reste R¹, die sich an direkt benachbarten Kohlenstoffatomen befinden, nicht gleichzeitig Wasserstoff und nicht gleichzeitig Methyl darstellen, und worin m und n die ganze Zahl 1 bedeuten (N,N-Dipropoxy-p-toluidin), mit von 1,0 bis 4,0 mol, bevorzugt von 1,25 bis 2,50 mol Propylenoxid (1,2-Epoxypropan) pro mol eingesetztes 4-Toluidin in Gegenwart eines Katalysators.

Das im erfindungsgemäßen Verfahren als Edukt eingesetzte N,N-Dipropoxy-p-toluidin, also die Verbindung der Formel (I), in der R¹ Wasserstoff oder Methyl bedeuten, wobei aber die Reste R¹, die sich an direkt benachbarten Kohlenstoffatomen befinden, nicht gleichzeitig Wasserstoff und nicht gleichzeitig Methyl darstellen, und worin m und n die ganze Zahl 1 bedeuten, kann dabei ein kommerziell erworbenes Produkt oder ein separat durch eigene Herstellung mit nachfolgender Isolierung erhaltenes Edukt oder ein durch eigene Herstellung ohne separate Isolierung erhaltenes Edukt sein. Im letzteren Fall wird das Edukt in dem Reaktionsgefäß hergestellt, in dem das erfindungsgemäße Verfahren durchgeführt wird.

Das erfindungsgemäße Verfahren wird bevorzugt bei Temperaturen von 80 bis 150 °C, bevorzugt von 100 bis 150 °C, durchgeführt. Niedrigere Temperaturen führen ggf. zu unvollständigen Umsetzungen und jedenfalls zu unwirtschaftlich langsamen Umsetzungen, die bei unangepassten Dosierraten des Propylenoxids einen erheblichen und damit gefährlichen Druckaufbau im Reaktor mit sich bringen. Dagegen können höhere Temperaturen - insbesondere solche oberhalb der Grenztemperatur Tₑₓₒ (gemäß TRAS 410) von 150 °C des N,N-Dipropoxy-p-toluidins - zu unkontrollierten exothermen Zersetzungen mit Druckaufbau führen.

In dem erfindungsgemäßen Verfahren werden bevorzugt als Katalysatoren Alkali- und Erdalkalimetallhydroxide, Alkali- und Erdalkalimetallcarbonate, Alkalimetalle, Lithiumalkyle, Natriumhydrid, komplexe Hydride wie Lithiumaluminiumhydrid, Natriumbis(methoxyethoxy)aluminiumdihydrid, oder Alkalimetallalkoholate eingesetzt. Besonders bevorzugt werden als Katalysatoren Alkalimetallhydroxide, Alkali- und Erdalkalimetallcarbonate, ganz besonders bevorzugt Natriumhydroxid oder Kaliumhydroxid verwendet. Pro mol Verbindung der Formel (I), in der R¹ Wasserstoff oder Methyl bedeuten, wobei aber die Reste R¹, die sich an direkt benachbarten Kohlenstoffatomen befinden, nicht gleichzeitig Wasserstoff und nicht gleichzeitig Methyl darstellen, und worin m und n die ganze Zahl 1 bedeuten (N,N-Dipropoxy-p-toluidin), werden bevorzugt 0,01 bis 0,05 mol Katalysator, besonders bevorzugt 0,02 bis 0,035 mol Katalysator, in das erfindungsgemäße Verfahren eingesetzt.

Die Bereitstellung des Eduktes N,N-Dipropoxy-p-toluidin, also der Verbindung der Formel (I), in der R¹ Wasserstoff oder Methyl bedeuten, wobei aber die Reste R¹, die sich an direkt benachbarten Kohlenstoffatomen befinden, nicht gleichzeitig Wasserstoff und nicht gleichzeitig Methyl darstellen, und worin m und n die ganze Zahl 1 bedeuten, für das erfindungsgemäße Verfahren kann durch Umsetzung von 4-Toluidin mit von 1,8 bis 2,2 mol, bevorzugt von 1,9 bis 2,1 mol, Propylenoxid pro eingesetztes mol 4-Toluidin, bei Temperaturen von 80 bis 150 °C, bevorzugt von 100 bis 150 °C, besonders bevorzugt von 110 bis 150 °C, in Abwesenheit von Katalysatoren erfolgen.

Damit umfasst das erfindungsgemäße Verfahren zur Herstellung der erfindungsgemäßen Mischungen auch die Umsetzung von 4-Toluidin mit von 1,8 bis 2,2, bevorzugt von 1,9 bis 2,1 mol Propylenoxid pro eingesetztes mol 4-Toluidin, bei Temperaturen von 80 bis 150 °C, bevorzugt von 100 bis 150 °C, besonders bevorzugt von 110 bis 150 °C, in Abwesenheit von Katalysatoren zur Bereitstellung des Eduktes, also der Verbindung der Formel (I), in der R¹ Wasserstoff oder Methyl bedeuten, wobei aber die Reste R¹, die sich an direkt benachbarten Kohlenstoffatomen befinden, nicht gleichzeitig Wasserstoff und nicht gleichzeitig Methyl darstellen, und worin m und n die ganze Zahl 1 bedeuten, mit nachfolgender Umsetzung des Eduktes mit von 1,0 bis 4,0 mol, bevorzugt von 1,25 bis 2,50 mol Propylenoxid, pro mol 4-eingesetztes Toluidin in Gegenwart eines Katalysators.

Das erfindungsgemäße Verfahren wird üblicherweise derart ausgeführt, dass vor Beginn der Umsetzung eine Temperatur gewählt wird, bei der die zu propoxylierende Komponente, also 4-Toluidin oder N,N-Dipropoxy-p-toluidin, in flüssiger Form vorliegt. Dann wird der Reaktor mit einem Inertgas, beispielsweise Stickstoff, inertisiert und nachfolgend der Innendruck auf einen Druck von ca. 50 bis 700 Hektopascal (hPa) reduziert. Bei der erfindungsgemäßen Propoxylierung von N,N-Dipropoxy-p-toluidin wird vorzugsweise vor dem Verschließen des Reaktors der Katalysator zugegeben. Die erfindungsgemäßen Katalysatoren werden bevorzugt in fester Form zugegeben, beispielsweise in Form von Schuppen, Perlen oder Pulver. Auch ein Einsatz als wässrige Lösung ist möglich, wobei das Wasser vor der weiteren Umsetzung herausdestilliert werden kann oder diese Entfernung des Wassers unterbleiben kann.

Bevorzugt wird nach der Umsetzung von 4-Toluidin oder N,N-Dipropoxy-p-toluidin noch im Reaktor befindliches Propylenoxid mit Inertgas ausgetrieben.

Üblicherweise wird die Umsetzung von 4-Toluidin oder N,N-Dipropoxy-4-toluidin mit Propylenoxid in einem geschlossenen druckdichten Reaktor, beispielsweise einem Autoklaven, durchgeführt. Bei der Reaktion werden ausgehend vom zuvor eingestellten Druck üblicherweise Druckanstiege im Bereich von 400 hPa bis 2000 hPa erreicht. Absolute Drücke im Reaktor von mehr als 0,3 Megapascal (MPa) werden üblicherweise nicht erreicht. Diese Drücke können jedoch deutlich übertroffen werden, wenn zu der zu propoxylierenden Komponente 4-Toluidin oder N,N-Dipropoxy-p-toluidin das gasförmige Propylenoxid entweder vor oder nach Erreichen der Reaktionstemperatur dem Reaktor vollständig zugegeben oder dem Reaktor vor oder nach Erreichen der Reaktionstemperatur zu schnell zudosiert wird. Beides ist in der Praxis aus sicherheitstechnischen Aspekten zu vermeiden. Üblicherweise wird das Propylenoxid dem Reaktionsgemisch, das aus der zu propoxylierenden Komponente 4-Toluidin oder N,N-Dipropoxy-p-toluidin und Propylenoxid entsteht, so zudosiert, dass ein Druck von ca. 0,2 MPa nicht überschritten wird.

Propylenoxid wird üblicherweise in kommerziell erhältlicher racemischer Form eingesetzt, das eine Reinheit von mindestens 99 % aufweist. In das erfindungsgemäße Verfahren können auch die einzelnen Enantiomeren, also R- und/oder S-Propylenoxid oder beliebige Mischungen davon eingesetzt werden.

Dabei wird die erfindungsgemäße Umsetzung von N,N-Dipropoxy-p-toluidin mit Propylenoxid immer mit Katalysator durchgeführt. Die Propoxylierung von 4-Toluidin zum N,N-Dipropoxy-p-toluidin wird dagegen in Abwesenheit von Katalysatoren durchgeführt.

Bevorzugt wird das erfindungsgemäße Verfahren in Abwesenheit von Lösungsmitteln durchgeführt.

In einer bevorzugten Ausführungsform des erfindungsgemäßen Verfahrens erfolgt die Bereitstellung des Eduktes N,N-Dipropoxy-p-toluidin durch Umsetzung eines 4-Toluidins, das einen Anteil von maximal 0,5 Gew.% 3-Toluidin, bezogen auf 4-Toluidin, aufweist. In einer alternativen bevorzugten Ausführungsform des erfindungsgemäßen Verfahrens erfolgt die Bereitstellung des Eduktes N,N-Dipropoxy-p-toluidin durch Umsetzung eines 4-Toluidins, das einen Anteil von maximal 0,2 Gew.% 3-Toluidin, bezogen auf 4-Toluidin, aufweist.

Um das spezielle, besonders reine 4-Toluidin mit begrenztem 3-Toluidin-Gehalt zu erhalten, muss man aus dem technischen 4-Toluidin das 3-Toluidin durch sorgfältige Destillation entfernen, da die Siedepunkte der beiden Isomere nahe beieinander liegen (Kp. von 4-Toluidin: 200,5 °C; Kp. von 3-Toluidin: 203,4 °C).

Eine weitere Möglichkeit, 4-Toluidin mit einem Gehalt an 3-Toluidin von maximal 0,2 Gew.% herzustellen, besteht darin, technisches N-Acetyl-4-toluidin umzukristallisieren, anschließend zu verseifen und zu destillieren. 4-Toluidin mit einem Gehalt an 3-Toluidin von weniger als 0,2 Gew.% wird in bevorzugter Weise hergestellt, indem man zunächst Toluol nitriert, aus dem dabei entstehenden Isomerengemisch von 2-, 3- und 4-Nitrotoluol die beiden unerwünschten Isomere 2- und 3-Nitrotoluol durch Optimierung des Rücklaufverhältnisses sehr gut bis zur erforderlichen Reinheit destillativ abtrennt und das erhaltene 4-Nitrotoluol anschließend einer Hydrierung unter Bildung des 4-Toluidins gewünschter Reinheit unterwirft. Auf diesem Weg kann 4-Toluidin mit einem Gehalt von weniger als 0,2 Gew.%, bevorzugt von weniger als 0,1 Gew.% 3-Toluidin auch in technischen Mengen zur Verfügung gestellt werden.

Die Herstellung der erfindungsgemäßen Mischungen kann ausgehend von 4-Toluidin nicht in einem Schritt erfolgen, indem entweder die gesamte erforderliche Menge an Propylenoxid und der Katalysator oder die gesamte erforderliche Menge an Propylenoxid ohne Katalysator zum vorgelegten 4-Toluidin zugegeben werden. Dabei entstehen in deutlichem Ausmaß unerwünschte Nebenprodukte und/oder das eingesetzte 4-Toluidin setzt sich nicht vollständig um.

Bei der Durchführung der bevorzugten Ausführungsformen des erfindungsgemäßen Verfahrens ist entscheidend, dass die engen Grenzen der Parameter bezüglich der molaren Verhältnisse zwischen Propylenoxid und 4-Toluidin und/oder zwischen Katalysator und 4-Toluidin und/oder der Reaktionstemperatur eingehalten werden um die gewünschte enge Verteilung der einzelnen Homologen des propoxylierten 4-Toluidins der Formel (I) zu erreichen. Da diese Parameter sich gegenseitig beeinflussen, kann die Kombination aus allen oberen oder aus allen unteren Grenzen der Parameter in Zusammenhang mit anderen Parametern, beispielsweise der Dosiergeschwindigkeit, der Vermischung der flüssigen Reaktionsphase aus 4-Toluidin, Katalysator und eindosiertem gasförmigem Propylenoxid dazu führen, dass in Einzelfällen eine nicht erfindungsgemäße Mischung aus propoxylierten 4-Toluidinen entsteht. Der Fachmann kann aber durch Anpassung dieser Parameter innerhalb dieser engen Grenzen je nach Versuchsaufbau oder Größe des Reaktionsgefäßes die geeignete Kombination der Parameter ohne besonderen Aufwand in einfacher Weise ermitteln, die innerhalb der vorgegebenen Grenzen zu den erfindungsgemäßem Mischungen führen.

Die Ausbeute der erfindungsgemäßen Propoxylierung ist praktisch quantitativ und wird nur durch Verluste in der Handhabung begrenzt, wie sie z.B. beim Umfüllen durch Anhaften von Restmengen an der Reaktorwand entstehen. Nach der beendeten Propoxylierung hat es sich bewährt, die Reaktionsmischung auf eine Temperatur im Bereich von 60 bis 100 °C abzukühlen und für einen gewissen Zeitraum Stickstoff durch die Reaktionsmischung zu leiten, um gegebenenfalls noch vorhandenes Propylenoxid vollständig aus dem System zu entfernen.

Das Reaktionsgemisch kann nach dem Fachmann bekannten Methoden aufgearbeitet oder aber auch unmittelbar weiter verwendet werden.

Die Verwendung von Propylenoxid als Propoxylierungsmittel bietet gegenüber anderen Reagenzien (wie z.B. 1-Chlorpropan-2-ol, 1-Brompropan-2-ol oder 1-Iodpropan-2-ol) den Vorteil, dass Hilfsreagenzien (wie stöchiometrische Mengen an Basen als Halogenwasserstoff-Fänger) nicht eingesetzt werden müssen und dementsprechend keine Salze gebildet werden, die in einem gesonderten Schritt abgetrennt werden müssten. Ein anderer Nachteil von der Verwendung von Halogenpropanolen ist beispielsweise Korrosion an den verwendeten Metallapparaturen.

Gegenstand der Erfindung ist ferner die Verwendung der erfindungsgemäßen Mischungen als Polymerisations- oder Vulkanisationsbeschleuniger, bevorzugt in der Polymerisation von Polyestern, insbesondere von ungesättigten Polyestern, oder als Härterkomponente für Epoxidharze. Es hat sich dabei bewährt, die erfindungsgemäße Mischung in einer Menge von 0,1 - 5 Gew.% einzusetzen. Die Polymerisation, in der die erfindungsgemäßen Mischungen eingesetzt werden können, ist bevorzugt eine radikalische Polymerisation.

Dabei weisen die erfindungsgemäßen Mischungen in den Polymerisationssystem, in denen das bekannte N,N-Dipropoxy-p-toluidin vorteilhaft gegenüber ethoxylierten oder anderen alkylierten 4-Toluidinen eingesetzt werden, eine verbesserte Handhabbarkeit und/oder eine bessere Verarbeitbarkeit und/oder eine geringere benötigte Aufwandmenge und/oder eine höhere Reaktivität auf. Der Ersatz des bekannten als feste Schmelze vorliegenden N,N-Dipropoxy-p-toluidins durch die erfindungsgemäßen flüssigen Mischungen in der radikalischen Polymerisation von Polyestern, insbesondere von ungesättigten Polyestern, oder als Härterkomponente für Epoxidharze kann vorteilhaft die physikochemischen und/oder physikomechanischen Eigenschaften der damit hergestellten Polymere beeinflussen.

In einer alternativen Ausführungsform können die erfindungsgemäßen Mischungen, die einen geringen Anteil von propoxylierten 3-Toluidinen enthalten, sich dadurch auszeichnen, dass sie besonders vorteilhaft als Polymerisations- oder Vulkanisationsbeschleuniger bei der Herstellung farbloser Polymere eingesetzt werden können, da ihre Verwendung zu keinerlei Verfärbungen des Polymers führt. Dies ist je nach gewünschter Anwendung des Polymers von hoher Bedeutung und nicht mit beliebigen Polymerisations- und Vulkanisationsbeschleunigern des Standes der Technik erreichbar.

Gegenstand der Erfindung ist auch ein polymeres Produkt erhältlich durch Polymerisation, bevorzugt eines Polyesters, insbesondere eines ungesättigten Polyesters, in Gegenwart der erfindungsgemäßen Mischungen als Polymerisations- oder Vulkanisationsbeschleuniger oder als Härterkomponente für Epoxidharze. Die Polymerisation, in der die erfindungsgemäßen Mischungen eingesetzt werden können, ist bevorzugt eine radikalische Polymerisation.

### Beispiele:

### Beispiele 1a bis 1e: Herstellung des propoxylierten Toluidins ausgehend von N,N-Dipropoxy-p-toluidin

### Beispiel 1a

In einem 3 Liter-Autoklaven (Edelstahl) mit Rührer, Innenthermometer, eintauchendem Einleitungsrohr für das Propylenoxid und Steigrohr zum Ausnehmen wurden 1425 g 98%iges N,N-Dipropoxy-p-toluidin [Verbindung der Formel (I), in der m und n jeweils die ganze Zahl 1 sind; 6,25 mol] als Schmelze und 10,9 g ca. 90%ige Kaliumhydroxid-Schuppen (0,175 mol) vorgelegt. Der Autoklav wurde verschlossen, durch Aufdrücken von Stickstoff inertisiert, entspannt und auf ca. 670 Hektopascal (hPa) (absoluter Druck) evakuiert. Der Inhalt wurde auf >80 °C aufgeheizt, um das N,N-Dipropoxy-p-toluidin weitgehend aufzuschmelzen. Dann wurde die Schmelze auf gewünschte Reaktionstemperatur (120 °C) aufgeheizt. Bei dieser Temperatur wurde die vorgesehene Menge Propylenoxid (hier 690,1 g = 11,88 mol, entsprechend 1,9 molare Äquivalente bezogen auf N,N-Dipropoxy-p-toluidin, also insgesamt 3,9 molare Äquivalente bezogen auf 4-Toluidin) in einer Geschwindigkeit von ca. 163 g/h zudosiert, wobei ein Druck von 0,18 MPa (absoluter Druck) kurzzeitig erreicht wurde. Dieser Druck wurde auch in den anderen Beispielen typischerweise nicht überschritten. Ca. 90 min. nach Ende der Dosierung ist der Gesamtdruck auf einen dann für ca. 15 min. konstanten Druck von ca. 800 hPa gefallen. Anschließend wurde noch weitere 60 min bei Reaktionstemperatur nachgerührt, dann auf 40 °C abgekühlt, der Unterdruck mit Stickstoff ausgeglichen, evtl. noch vorhandenes Propylenoxid mit Stickstoff ausgeblasen und der Ansatz über einen Klärfilter abgefüllt. Es wurden 2109,3 g (Ausbeute: 99,2% der Einsatzmengen) Produkt erhalten, das folgende Verteilung (in Gewichtsprozenten) der Homologen der Formel (I) aufweist:

| | m = 1; n = 1 | m = 1; n = 2 | m + n = 4 | m + n = 5 | m + n > 5 |
|---|---|---|---|---|---|
| Gehalt Verbindung der Formel (I) | 0,51 Gew.% | 41,7 Gew.% | 35,6 Gew.% | 14,6 Gew.% | 6,27 Gew.% |

Die Beispiele 1b bis 1e wurden analog durchgeführt - ggf. in einem 0,5 Liter-Autoklaven. Anstatt der vorgegebenen Mengen in Beispiel 1a wurden die Beispiele 1b bis 1e mit den in der Tabelle 1 angegebenen Daten durchgeführt.

**Tabelle 1: Reaktionsdaten der Beispiele 1b bis 1e**

| **Reaktionsdaten** | **Beispiel 1b** | **Beispiel 1c** | **Beispiel 1d** | **Beispiel 1e** |
|---|---|---|---|---|
| | erfindungsgemäß | erfindungsgemäß | erfindungsgemäß | nicht erfindungsgemäß |
| Autoklavengröße | 3 L | 3 L | 0,5 L | 0,5 L |
| 98%iges N,N-Dipropoxy-p-toluidin | 1425 g | 1425 g | 226 g | 226 g |
| | 6,25 mol | 6,25 mol | 1,00 mol | 1,00 mol |
| Katalysator: | 99,8%ige Natriumhydroxid- | 99,8%ige Natriumhydroxid- | 90%ige Kaliumhydroxid- | 90%ige Kaliumhydroxid- |

| | Perlen | Perlen | Schuppen | Schuppen |
|---|---|---|---|---|
| Menge Katalysator | 5,73 g | 5,73 g | 1,13g | 1,13 g |
| | 0,143 mol | 0,143 mol | 0,018 mol | 0,018 mol |
| | 2,3 mol% ¹⁾ | 2,3 mol% ¹⁾ | 1,8 mol% ¹⁾ | 1,8 mol% ¹⁾ |
| Reaktionstemperatur | 120 °C | 120 °C | 120 °C | 120 °C |
| Propylenoxid | 671,9 g | 635,6 g | 58,2 g | 29,1 g |
| | 11,56 mol | 10,94 mol | 1,00 mol | 0,50 mol |
| | 1,85 moleq.²⁾ | 1,75 moleq.²⁾ | 1,00 moleq.²⁾ | 0,50 moleq.²⁾ |
| Ausbeute | 2047 g | 2012 g | 278 g | 250 g |
| | 97,4% der eingesetzten | 97,4% der eingesetzten | 97,4% der eingesetzten | 97,6% der eingesetzten |
| | Mengen | Mengen | Mengen | Mengen |
| Gehalt Verbindung der Formel (I) | | | | |
| m = 1; n = 1 | 0,95 Gew.% | 1,31 Gew.% | 11,0 Gew.% | 47,4 Gew.% |
| m = 1; n = 2 | 44,6Gew.% | 47,9 Gew.% | 72,2 Gew.% | 41,9 Gew.% |
| m + n = 4 | 32,0 Gew.% | 31,0 Gew.% | 13,0 Gew.% | 10,5 Gew.% |
| m + n = 5 | 14,0 | 12,4 Gew.% | 3,65 Gew.% | <0,1 Gew.% |
| m+n > 5 | 7,66 | 6,20 Gew.% | <0,1 Gew.% | <0,1 Gew.% |
| Aggregatzustand bei 20 °C | flüssig, leicht viskos | flüssig, leicht viskos | flüssig, viskos | stark viskos, überwiegend mit festen Anteilen |
| Aggregatzustand bei 5 °C | deutlich viskos | deutlich viskos | hochviskos | fest mit glasartig erstarrten Anteilen |
| Aggregatzustand bei -15 °C | glasartig erstarrt | glasartig erstarrt | glasartig erstarrt | fest mit glasartig erstarrten Anteilen |

| | | | | |
|---|---|---|---|---|
| ¹⁾ 1 mol%.: 0,01 mol Base bezogen auf 1 mol eingesetztes N,N-Dipropoxy-p-toluidin ²⁾ x moleq.: x mol Propylenoxid bezogen auf 1 mol eingesetztes N,N-Dipropoxy-p-toluidin | | | | |

### Beispiele 2a bis 2e: Herstellung des propoxylierten Toluidins ausgehend von 4-Toluidin

### Beispiel 2a

In einem 3 Liter-Autoklav (Edelstahl) mit Rührer, Innenthermometer, eintauchendem Einleitungsrohr für das Propylenoxid und Steigrohr zum Ausnehmen wurden 672,1 g 99,7%iges 4-Toluidin (6,25 mol) als Schmelze vorgelegt. Der Autoklav wurde verschlossen, durch Aufdrücken von Stickstoff inertisiert, entspannt und auf ca. 670 hPa (abs.) evakuiert. Der Inhalt wurde zunächst ohne zu rühren auf >45 °C aufgeheizt, um das 4-Toluidin vollständig aufzuschmelzen. Dann wurde die Schmelze weiter auf die gewünschte Reaktionstemperatur (120 °C) aufgeheizt. Bei dieser Temperatur wurden in Abwesenheit von Katalysatoren 726,4 g Propylenoxid (12,49 mol) in einer Geschwindigkeit von ca. 163 g/h zudosiert, wobei ein Druck von maximal 0,27 MPa (abs.) kurzzeitig erreicht wurde. Dieser Druck wurde auch in den anderen Beispielen typischerweise nicht überschritten. Ca. 3,5 h nach Ende der Dosierung ist der Gesamtdruck auf einen dann für ca. 15 min. konstanten Druck von ca. 770 hPa gefallen. Anschließend wurde noch weitere 60 min. bei Reaktionstemperatur nachgerührt, auf 80 bis 100 °C abgekühlt, der Unterdruck mit Stickstoff ausgeglichen. Mittels Probenahme kann überprüft werden, ob die typische Zusammensetzung von N,N-Dipropoxy-p-toluidin erreicht wurde.

Anschließend wurde die vorgesehene Menge (2,8 mol% bezogen auf eingesetztes 4-Toluidin) an festem ca. 90%igem Kaliumhydroxid zugesetzt, der Autoklav wieder verschlossen, wie oben beschrieben inertisiert, evakuiert und auf die gewünschte Reaktionstemperatur von 120 °C aufgeheizt. Anschließend wurden weitere 690,1 g (11,88 mol) Propylenoxid mit ca. 163 g/h zudosiert, wobei gegen Ende ein Druckanstieg um ca. 760 hPa auf ca. 0,143 MPa (absolut) beobachtet wurde. Ca. 50 min. nach Ende der Dosierung ist der Gesamtdruck auf den ursprünglichen Druck von 670 hPa gefallen. Anschließend wurde noch weitere 60 min bei Reaktionstemperatur nachgerührt, dann auf 40 °C abgekühlt, der Unterdruck mit Stickstoff ausgeglichen, evtl. noch vorhandenes Propylenoxid mit Stickstoff ausgeblasen und der Ansatz über einen Klärfilter abgefüllt. Es wurden 2087,4 g (Ausbeute: 99,4% der eingesetzten Mengen) Produkt erhalten, das folgende Verteilung (in Gewichtsprozenten) der Homologen der Formel (I) enthält:

| | m = 1; n = 1 | m = 1; n = 2 | m + n = 4 | m + n = 5 | m + n > 5 |
|---|---|---|---|---|---|
| Gehalt Verbindung der Formel (I) | 1,69 Gew.% | 38,9 Gew.% | 35,2 Gew.% | 15,6 Gew.% | 7,00 Gew.% |

Die Beispiele 2b bis 2e wurden analog durchgeführt - ggf. in einem 0,5L-Autoklaven. Anstatt der vorgegebenen Mengen in Beispiel 2a wurden die Beispiele 2b bis e mit den in der Tabelle 2 angegebenen Daten durchgeführt.

**Tabelle 2: Reaktionsdaten der Beispiele 2b bis 2e**

| **Reaktionsdaten** | **Beispiel 2b** | **Beispiel 2c** | **Beispiel 2d** | **Beispiel 2e** |
|---|---|---|---|---|
| | erfindungsgemäß | erfindungsgemäß | erfindungsgemäß | nicht erfindungsgemäß |
| Autoklavengröße | 0,5 L | 0,5 L | 0,5 L | 0,5 L |
| 99,7%iges 4-Toluidin | 107,5 g | 107,5 g | 107,5 g | 77,4 g |
| | 1 mol | 1 mol | 1 mol | 0,72 mol |

| 1. Stufe | | | | |
|---|---|---|---|---|
| Propylenoxid | 116,2 g | 116,2 g | 116,2 g | 83,6 g |
| | 2 mol | 2 mol | 2 mol | 1,44 mol |
| | 2,0 moleq.²⁾ | 2,0 moleq.²⁾ | 2,0 moleq.²⁾ | 2,0 moleq.²⁾ |
| Reaktionstemperatur | 110 °C | 130 °C | 120 °C | 120 °C |

| **2. Stufe** | | | | |
|---|---|---|---|---|
| Katalysator: | 90%ige Kaliumhydroxid-Schuppen | 90%ige Kaliumhydroxid-Schuppen | 90%ige Kaliumhydroxid-Schuppen | 90%ige Kaliumhydroxid-Schuppen |
| Menge Katalysator | 1,75 g | 1,75 g | 1,75 g | 1,26 g |
| | 0,028 mol | 0,028 mol | 0,028 mol | 0,020 mol |
| | 2,8 mol% ¹⁾ | 2,8 mol% ¹⁾ | 2,8 mol% ¹⁾ | 2,8 mol% ¹⁾ |
| Reaktionstemperatur | 110 °C | 130 °C | 120 °C | 120 °C |
| Propylenoxid | 101,6 g | 101,6 g | 58,1 g | 177,8 g |
| | 1,75 mol | 1,75 mol | 1,0 mol | 3,06 mol |
| | 1,75 moleq.²⁾ | 1,75 moleq.²⁾ | 1,0 moleq.²⁾ | 4,25 moleq.²⁾ |
| Ausbeute | 317,5 g | 317,9 g | 266,3 g | 316 g |
| | 97,1% der eingesetzten | 97,1% der eingesetzten | 93,9% der eingesetzten | 92,9% der eingesetzten |
| | Mengen | Mengen | Mengen | Mengen |

| Gehalt Verbindung der Formel (I) | | | | |
|---|---|---|---|---|
| m = 1; n = 1 | 2,27 Gew.% | 1,08 Gew.% | 12,9 Gew.% | 0,55 Gew.% |
| m = 1; n = 2 | 47,8 Gew.% | 33,5 Gew.% | 74,9 Gew.% | 5,36 Gew.% |
| m + n = 4 | 32,7 Gew.% | 35,3 Gew.% | 8,71 Gew.% | 16,0 Gew.% |
| m + n = 5 | 11,3 Gew. % | 18,2 Gew.% | 0,39 Gew.% | 20,8 Gew.% |
| m + n > 5 | 3,79 Gew. % | 10,2 Gew.% | 0,20 Gew.% | 56,3 Gew.% |
| Aggregatzustand bei 20 °C | flüssig, leicht viskos | flüssig, leicht viskos | flüssig, viskos | flüssig, leicht viskos |
| Aggregatzustand bei 5 °C | deutlich viskos | deutlich viskos | hochviskos | deutlich viskos |
| Aggregatzustand bei -15 °C | glasartig erstarrt | glasartig erstarrt | glasartig erstarrt | glasartig erstarrt |

| | | | | |
|---|---|---|---|---|
| ¹⁾ 1 mol%.: 0,01 mol Base bezogen auf 1 mol eingesetztes 4-Toluidin ²⁾ x moleq.: x mol Propylenoxid bezogen auf 1 mol eingesetztes 4-Toluidin | | | | |

## Patentansprüche

1. Mischungen enthaltend zwei oder mehr verschiedene Verbindungen der allgemeinen Formel (I), 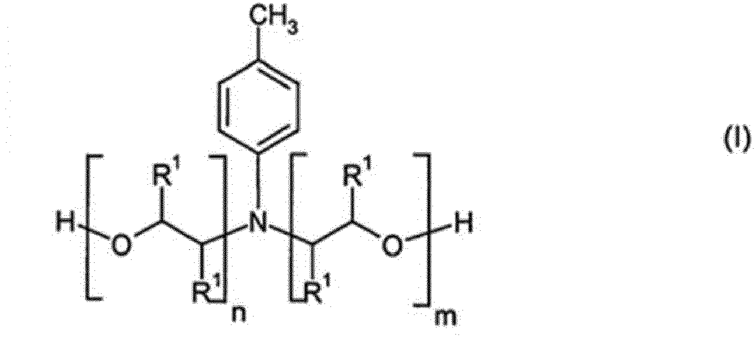
worin R¹ Wasserstoff oder Methyl bedeutet, wobei aber die Reste R¹, die sich an direkt benachbarten Kohlenstoffatomen befinden, nicht gleichzeitig Wasserstoff und nicht gleichzeitig Methyl und m und n ganze Zahlen darstellen, **dadurch gekennzeichnet, dass**
4-Toluidin in einem Anteil von maximal 2 Gew.%, bevorzugt von 0,001 bis 1 Gew.%, bezogen auf die gesamte Masse aller Verbindungen der Formel (I) in der Mischung vorliegt, und
die Verbindungen der Formel (I), bei denen die Summe aus m und n die ganze Zahl 2 beträgt, in einem Anteil von maximal 20 Gew.%, bevorzugt von 0,01 bis 20 Gew.%, besonders bevorzugt von 0,01 bis 12 Gew.%, bezogen auf die gesamte Masse aller Verbindungen der Formel (I) in der Mischung vorliegen, und
die Verbindungen der Formel (I), bei denen die Summe aus m und n mindestens die ganze Zahl 6 beträgt ist, in einem Anteil von maximal 40 Gew.%, bevorzugt von 0,01 bis 40 Gew.%, besonders bevorzugt von 0,01 bis 20 Gew.%, bezogen auf die gesamte Masse aller Verbindungen der Formel (I) in der Mischung vorliegen.

2. Mischungen nach Anspruch 1, **dadurch gekennzeichnet, dass** die Verbindungen der Formel (I), bei denen die Summe aus m und n die ganze Zahl 3 beträgt, in einem Anteil von 7 bis 49 Gew.%, bevorzugt von 15 bis 49 Gew.%, bezogen auf die gesamte Masse aller Verbindungen der Formel (I) in der Mischung vorliegen.

3. Mischungen nach Anspruch 1 oder 2, **dadurch gekennzeichnet, dass** die Verbindung der Formel (I), bei denen die Summe aus m und n die ganze Zahl 4 beträgt, in einem Anteil von 10 bis 49 Gew.%, bevorzugt von 10 bis 40 Gew.%, bezogen auf die gesamte Masse aller Verbindungen der Formel (I) in der Mischung vorliegt.

4. Mischungen nach einem der Ansprüche 1 bis 3, **dadurch gekennzeichnet, dass** jede homologe Gruppe von Verbindungen der Formel (I) in der Mischung in einem Anteil von kleiner als 50 Gewichtsprozenten bezogen auf die gesamte Masse aller Verbindungen der Formel (I) in der Mischung enthalten ist.

5. Mischungen nach einem der Ansprüche 1 bis 4, **dadurch gekennzeichnet, dass** sie einen Anteil an 4-Toluidin von weniger als 0,1 Gew.% bezogen auf die gesamte Masse aller Verbindungen der Formel (I) in der Mischung aufweisen.

6. Mischungen nach einem der Ansprüche 1 bis 5, **dadurch gekennzeichnet, dass** sie bei Temperaturen von 5 bis 40 °C einen flüssigen Aggregatzustand aufweisen.

7. Mischungen nach einem der Ansprüche 1 bis 6, **dadurch gekennzeichnet, dass** sie eine dynamische Viskosität von 500 bis 20000 mPas bei einer Temperatur von 25 °C, gemessen gemäß DIN 53019 mit einem Rotationsviskosimeter, aufweisen.

8. Mischungen nach einem der Ansprüche 1 bis 7, **dadurch gekennzeichnet, dass** sie von 96 bis 100 Gew.% Verbindungen der Formel (I) enthalten.

9. Verfahren zur Herstellung der Mischungen gemäß einem der Ansprüche 1 bis 8, umfassend die Umsetzung der Verbindungen der Formel (I), in der R¹ Wasserstoff oder Methyl bedeuten, wobei aber die Reste R¹, die sich an direkt benachbarten Kohlenstoffatomen befinden, nicht gleichzeitig Wasserstoff und nicht gleichzeitig Methyl darstellen, und worin m und n die ganze Zahl 1 bedeuten, mit von 1,0 bis 4,0 mol, bevorzugt von 1,25 bis 2,50 mol Propylenoxid, pro mol eingesetztes 4-Toluidin in Gegenwart eines Katalysators.

10. Verfahren zur Herstellung der Mischungen gemäß Anspruch 9, wobei die Umsetzung bei Temperaturen von 80 bis 150 °C, bevorzugt von 100 bis 150 °C, erfolgt.

11. Verfahren zur Herstellung der Mischungen gemäß Anspruch 9 oder 10, wobei die Umsetzung in Gegenwart von 0,01 bis 0,05 mol Katalysator, ausgewählt aus Alkali- und Erdalkalimetallhydroxiden, Alkalimetallen, Lithiumalkylen, Natriumhydrid, komplexen Hydriden wie Lithiumaluminiumhydrid, Natriumbis(methoxyethoxy)aluminiumdihydrid, oder Alkalimetallalkoholate, bevorzugt ausgewählt aus Alkalimetallhydroxiden, Alkali- und Erdalkalimetallcarbonaten, pro mol eingesetzte Verbindung der Formel (I), in der R¹ Wasserstoff oder Methyl bedeuten, wobei aber die Reste R¹, die sich an direkt benachbarten Kohlenstoffatomen befinden, nicht gleichzeitig Wasserstoff und nicht gleichzeitig Methyl darstellen, und worin m und n die ganze Zahl 1 bedeuten (N,N-Dipropoxy-p-toluidin), erfolgt.

12. Verfahren zur Herstellung der Mischungen gemäß Anspruch 9, umfassend die Herstellung der Verbindung der Formel (I), in der R¹ Wasserstoff oder Methyl bedeuten, wobei aber die Reste R¹, die sich an direkt benachbarten Kohlenstoffatomen befinden, nicht gleichzeitig Wasserstoff und nicht gleichzeitig Methyl darstellen, und worin m und n die ganze Zahl 1 bedeuten (N,N-Dipropoxy-p-toluidin), durch Umsetzung von 4-Toluidin, mit von 1,8 bis 2,2 mol, bevorzugt von 1,9 bis 2,1 mol, Propylenoxid pro eingesetztes mol 4-Toluidin, bei Temperaturen von 80 bis 150 °C, bevorzugt von 100 bis 150 °C, besonders bevorzugt von 110 bis 150 °C, in Abwesenheit von Katalysatoren.

13. Verfahren zur Herstellung der Mischungen gemäß einem der Ansprüche 9 bis 12, **dadurch gekennzeichnet, dass** die Umsetzung in Abwesenheit von Lösungsmitteln stattfindet.

14. Verfahren zur Herstellung der Mischungen gemäß einem der Ansprüche 9 bis 13, wobei das 4-Toluidin einen Anteil von maximal 0,2 Gew.% 3-Toluidin, bezogen auf 4-Toluidin, aufweist.

15. Verwendung der Mischungen nach einem der Ansprüche 1 bis 8 als Polymerisations- oder Vulkanisationsbeschleuniger, bevorzugt in der radikalischen Polymerisation, bevorzugt von Polyestern, insbesondere von ungesättigten Polyestern, oder als Härterkomponente für Epoxidharze.

16. Polymeres Produkt erhältlich durch Polymerisation, bevorzugt eines Polyesters, insbesondere eines ungesättigten Polyesters, in Gegenwart von Mischungen nach einem der Ansprüche 1 bis 8 als Polymerisations- oder Vulkanisationsbeschleuniger oder als Härterkomponente für Epoxidharze.

## Claims

1. Mixtures comprising two or more different compounds of the general formula (I), 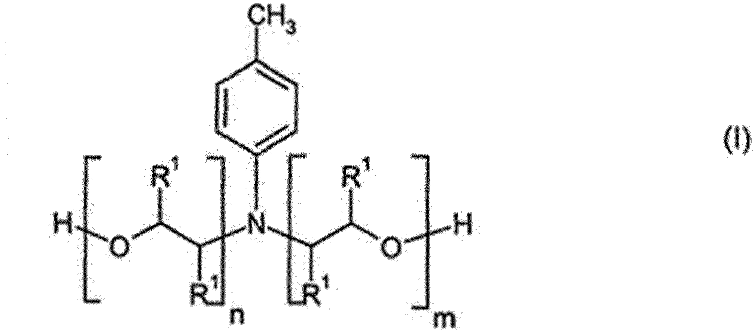
where R¹ is hydrogen or methyl, but where the R¹ radicals on directly adjacent carbon atoms are not simultaneously hydrogen and not simultaneously methyl, and m and n are integers, **characterized in that**
4-toluidine is present at a proportion of at maximum 2% by weight, preferably from 0.001 to 1% by weight, based on the total mass of all compounds of the formula (I) in the mixture, and
the compounds of the formula (I), in which the sum of m and n is the integer 2, are present at a proportion of at maximum 20% by weight, preferably from 0.01 to 20% by weight, particularly preferably from 0.01 to 12% by weight, based on the total mass of all compounds of the formula (I) in the mixture, and
the compounds of the formula (I), in which the sum of m and n is at least the integer 6, are present at a proportion of at maximum 40% by weight, preferably from 0.01 to 40% by weight, particularly preferably from 0.01 to 20% by weight, based on the total mass of all compounds of the formula (I) in the mixture.

2. Mixtures according to Claim 1, **characterized in that** the compounds of the formula (I) in which the sum of m and n is the integer 3 are present at a proportion of 7 to 49% by weight, preferably from 15 to 49% by weight, based on the total mass of all compounds of the formula (I) in the mixture.

3. Mixtures according to Claim 1 or 2, **characterized in that** the compound of the formula (I) in which the sum of m and n is the integer 4 is present at a proportion of 10 to 49% by weight, preferably from 10 to 40% by weight, based on the total mass of all compounds of the formula (I) in the mixture.

4. Mixtures according to any of Claims 1 to 3, **characterized in that** each homologous group of compounds of the formula (I) in the mixture is present at a proportion of less than 50 percent by weight, based on the total mass of all compounds of the formula (I) in the mixture.

5. Mixtures according to any of Claims 1 to 4, **characterized in that** said mixtures have a proportion of 4-toluidine of less than 0.1% by weight, based on the total mass of all compounds of the formula (I) in the mixture.

6. Mixtures according to any of Claims 1 to 5, **characterized in that** said mixtures have a liquid physical state at temperatures of 5 to 40°C.

7. Mixtures according to any of Claims 1 to 6, **characterized in that** said mixtures have a dynamic viscosity of 500 to 20 000 mPas at a temperature of 25°C, measured in accordance with DIN 53019 using a rotational viscometer.

8. Mixtures according to any of Claims 1 to 7, **characterized in that** said mixtures comprise from 96 to 100% by weight of compounds of the formula (I) .

9. Method for preparing the mixtures according to any of Claims 1 to 8, comprising reacting the compounds of the formula (I), in which R¹ are hydrogen or methyl, but where the R¹ radicals on directly adjacent carbon atoms are not simultaneously hydrogen and not simultaneously methyl, and in which m and n are the integer 1, with from 1.0 to 4.0 mol, preferably from 1.25 to 2.50 mol of propylene oxide, per mole of 4-toluidine used, in the presence of a catalyst.

10. Method for preparing the mixtures according to Claim 9, wherein the reaction is conducted at temperatures of 80 to 150°C, preferably 100 to 150°C.

11. Method for preparing the mixtures according to Claim 9 or 10, wherein the reaction is carried out in the presence of 0.01 to 0.05 mol of catalyst, selected from alkali metal hydroxides and alkaline earth metal hydroxides, alkali metals, lithium alkyls, sodium hydride, complex hydrides such as lithium aluminium hydride, sodium bis(methoxyethoxy)aluminium dihydride, or alkali metal alkoxides, preferably selected from alkali metal hydroxides, alkali metal carbonates and alkaline earth metal carbonates, per mole of compound of the formula (I) used, in which R¹ are hydrogen or methyl, but where the R¹ radicals on directly adjacent carbon atoms are not simultaneously hydrogen and not simultaneously methyl, and in which m and n are the integer 1 (N,N-dipropoxy-p-toluidine).

12. Method for preparing the mixtures according to Claim 9, comprising the preparation of the compound of the formula (I), in which R¹ are hydrogen or methyl, but where the R¹ radicals on directly adjacent carbon atoms are not simultaneously hydrogen and not simultaneously methyl, and in which m and n are the integer 1 (N,N-dipropoxy-p-toluidine), by reacting 4-toluidine with 1.8 to 2.2 mol, preferably 1.9 to 2.1 mol of propylene oxide per mole of 4-toluidine used, at temperatures of 80 to 150°C, preferably 100 to 150°C, particularly preferably 110 to 150°C, in the absence of catalysts.

13. Method for preparing the mixtures according to any of Claims 9 to 12, **characterized in that** the reaction is carried out in the absence of solvents.

14. Method for preparing the mixtures according to any of Claims 9 to 13, wherein the 4-toluidine has a proportion of at maximum 0.2% by weight of 3-toluidine, based on 4-toluidine.

15. Use of the mixtures according to any of Claims 1 to 8 as polymerization or vulcanization accelerators, preferably in radical polymerization, preferably of polyesters, especially of unsaturated polyesters, or as a curing component for epoxy resins.

16. Polymeric product obtainable by polymerization, preferably of a polyester, in particular of an unsaturated polyester, in the presence of mixtures according to one of Claims 1 to 8 as polymerization or vulcanization accelerators or as a curing component for epoxy resins.

## Revendications

1. Mélanges contenant deux composés différents ou plus de formule générale (I), 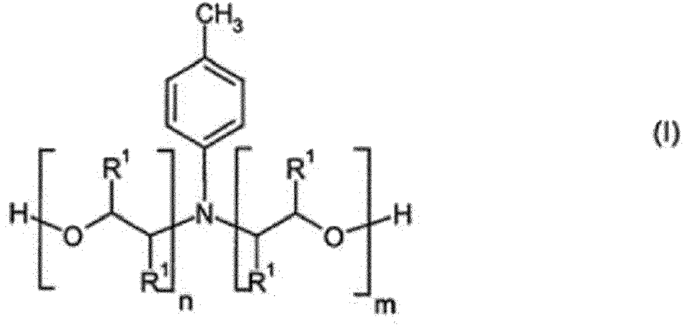
dans laquelle R¹ signifie hydrogène ou méthyle, mais les radicaux R¹ qui se trouvent au niveau d'atomes de carbone directement adjacents ne représentant pas en même temps hydrogène et pas en même temps méthyle et m et n représentant des nombres entiers, **caractérisés en ce que** la 4-toluidine est présente en une proportion de maximum 2 % en poids, préférablement de 0,001 à 1 % en poids, par rapport à la masse totale de tous les composés de formule (I) dans le mélange, et
les composés de formule (I), pour lesquels la somme de m et n est le nombre entier 2 sont présents en une proportion de maximum 20 % en poids, préférablement de 0,01 à 20 % en poids, particulièrement préférablement de 0,01 à 12 % en poids, par rapport à la masse totale de tous les composés de formule (I) dans le mélange, et les composés de formule (I), pour lesquels la somme de m et n est au moins le nombre entier 6 sont présents en une proportion de maximum 40 % en poids, préférablement de 0,01 à 40 % en poids, particulièrement préférablement de 0,01 à 20 % en poids, par rapport à la masse totale de tous les composés de formule (I) dans le mélange.

2. Mélanges selon la revendication 1, **caractérisés en ce que** les composés de formule (I), pour lesquels la somme de m et n est le nombre entier 3 sont présents en une proportion de 7 à 49 % en poids, préférablement de 15 à 49 % en poids, par rapport à la masse totale de tous les composés de formule (I) dans le mélange.

3. Mélanges selon la revendication 1 ou 2, **caractérisés en ce que** les composés de formule (I), pour lesquels la somme de m et n est le nombre entier 4 sont présents en une proportion de 10 à 49 % en poids, préférablement de 10 à 40 % en poids, par rapport à la masse totale de tous les composés de formule (I) dans le mélange.

4. Mélanges selon l'une quelconque des revendications 1 à 3, **caractérisés en ce que** chaque groupe homologue de composés de formule (I) dans le mélange est présent en une proportion inférieure à 50 pour cent en poids par rapport à la masse totale de tous les composés de formule (I) dans le mélange.

5. Mélanges selon l'une quelconque des revendications 1 à 4, **caractérisés en ce qu'**ils présentent une proportion en 4-toluidine inférieure à 0,1 % en poids, par rapport à la masse totale de tous les composés de formule (I) dans le mélange.

6. Mélanges selon l'une quelconque des revendications 1 à 5, **caractérisés en ce qu'**ils présentent un état d'agrégat liquide à des températures de 5 à 40 °C.

7. Mélanges selon l'une quelconque des revendications 1 à 6, **caractérisés en ce qu'**ils présentent une viscosité dynamique de 500 à 20 000 mPas à une température de 25 °C, mesurée selon la norme DIN 53019 avec un viscosimètre rotatif.

8. Mélanges selon l'une quelconque des revendications 1 à 7, **caractérisés en ce qu'**ils contiennent de 96 à 100 % en poids de composés de formule (I).

9. Procédé de préparation des mélanges selon l'une quelconque des revendications 1 à 8, comprenant la transformation des composés de formule (I), dans laquelle R¹ signifie hydrogène ou méthyle, mais les radicaux R¹ qui se trouvent au niveau d'atomes de carbone directement adjacents ne représentant pas en même temps hydrogène et pas en même temps méthyle et dans laquelle m et n signifient le nombre entier 1, avec de 1,0 à 4,0 moles, préférablement de 1,25 à 2,50 moles d'oxyde de propylène, par mole de 4-toluidine utilisée en présence d'un catalyseur.

10. Procédé de préparation des mélanges selon la revendication 9, la transformation étant réalisée à des températures de 80 à 150 °C, préférablement de 100 à 150 °C.

11. Procédé de préparation des mélanges selon la revendication 9 ou 10, la transformation étant réalisée en présence de 0,01 à 0,05 mole de catalyseur, choisi parmi des hydroxydes de métal alcalin et alcalino-terreux, des métaux alcalin, des alkyllithium, l'hydrure de sodium, des hydrures complexes comme l'hydrure de lithium et d'aluminium, le dihydrure de sodium bis(méthoxyéthoxy)aluminium, ou des alcoolates de métal alcalin, préférablement choisi parmi des hydroxydes de métal alcalin, des carbonates de métal alcalin et alcalino-terreux, par mole de composé de formule (I) utilisé, dans laquelle R¹ signifie hydrogène ou méthyle, mais les radicaux R¹ qui se trouvent au niveau d'atomes de carbone directement adjacents ne représentant pas en même temps hydrogène et pas en même temps méthyle et dans laquelle m et n signifient le nombre entier 1 (N,N-dipropoxy-p-toluidine).

12. Procédé de préparation des mélanges selon la revendication 9, comprenant la préparation du composé de formule (I), dans laquelle R¹ signifie hydrogène ou méthyle, mais les radicaux R¹ qui se trouvent au niveau d'atomes de carbone directement adjacents ne représentant pas en même temps hydrogène et pas en même temps méthyle et dans laquelle m et n signifient le nombre entier 1 (N,N-dipropoxy-p-toluidine), par transformation de 4-toluidine, avec de 1,8 à 2,2 moles, préférablement de 1,9 à 2,1 moles, d'oxyde de propylène par mole de 4-toluidine utilisée, à des températures de 80 à 150 °C, préférablement de 100 à 150 °C, particulièrement préférablement de 110 à 150 °C, en l'absence de catalyseurs.

13. Procédé de préparation des mélanges selon l'une quelconque des revendications 9 à 12, **caractérisé en ce que** la transformation a lieu en l'absence de solvants.

14. Procédé de préparation des mélanges selon l'une quelconque des revendications 9 à 13, la 4-toluidine présentant une proportion de maximum 0,2 % en poids de 3-toluidine, par rapport à la 4-toluidine.

15. Utilisation des mélanges selon l'une quelconque des revendications 1 à 8 en tant qu'accélérateur de polymérisation ou de vulcanisation, préférablement dans la polymérisation radicalaire, préférablement de polyesters, en particulier de polyesters insaturés, ou en tant que composant de durcissement pour des résines époxy.

16. Produit polymérique obtenu par polymérisation, préférablement d'un polyester, en particulier d'un polyester insaturé, en présence de mélanges selon l'une quelconque des revendications 1 à 8 en tant qu'accélérateur de polymérisation ou de vulcanisation ou en tant que composant de durcissement pour des résines époxy.
